# NEW EUROPEAN PATENT SPECIFICATION

(11) **EP 2 726 602 B2**
(45) Date of publication and mention of the opposition decision: **30.05.2018**
(45) Mention of the grant of the patent: 22.10.2014
(21) Application number: 12777875.1
(22) Date of filing: 28.09.2012
(51) Int. Cl.: C12N 5/071

(54) **HIGH-SAFETY PROCESS FOR THE PREPARATION OF PURIFIED STEM CELL FRACTIONS**
HOCHSICHERHEITSVERFAHREN ZUR HERSTELLUNG GEREINIGTER STAMMZELLFRAKTIONEN
PROCÉDÉ À HAUTE SÉCURITÉ POUR LA PRÉPARATION DE FRACTIONS DE CELLULES SOUCHES PURIFIÉES

(43) Date of publication of application: 07.05.2014
(73) Proprietor: SWISS STEM CELL FOUNDATION, 6925 Gentilino (CH)
(72) Inventor: SOLDATI, Gianni, 6968 Sonvico (CH); TALLONE, Tiziano, 6900 Lugano (CH)
(74) Representative: Zardi, Marco
(86) International application number: PCT/EP2012/069261
(87) International publication number: WO 2014/048501

(56) References cited:
- US-A1- 2005 048 036
- SINAN GÜVEN ET AL: "Validation of an Automated Procedure to Isolate Human Adipose Tissue-Derived Cells by Using the Sepax Technology", TISSUE ENGINEERING PART C: METHODS, vol. 18, no. 8, 30 March 2012 (2012-03-30) , pages 575-582, XP055065749, ISSN: 1937-3384, DOI: 10.1089/ten.tec.2011.0617
- YARAK SAMIRA ET AL: "Human adipose-derived stem cells: current challenges and clinical perspectives.", ANAIS BRASILEIROS DE DERMATOLOGIA 2010 SEP-OCT, vol. 85, no. 5, September 2010 (2010-09), pages 647-656, XP002698474, ISSN: 1806-4841
- TIZIANO TALLONE ET AL: "Adult Human Adipose Tissue Contains Several Types of Multipotent Cells", JOURNAL OF CARDIOVASCULAR TRANSLATIONAL RESEARCH, vol. 4, no. 2, 15 February 2011 (2011-02-15), pages 200-210, XP055065540, ISSN: 1937-5387, DOI: 10.1007/s12265-011-9257-3
- GIMBLE JEFFREY M ET AL: "Adipose-derived stem cells for regenerative medicine", CIRCULATION RESEARCH, GRUNE AND STRATTON, BALTIMORE, US, vol. 100, no. 9, 11 May 2007 (2007-05-11), pages 1249-1260, XP009089514, ISSN: 0009-7330
- Tesi di Laurea of Grazia Aita entitled "Induzione del defferenziamento osseo a scopo clinico e caratterizzazione dell'espressione genica di cellule mesenchimali umane su scaffold biocompatibile", available for inspection at Università degli Studi dell'Insubria, Varese, Italy since 23.03.2010
- Partial translation of D1 into English
- Declaration of Grazia Aita authorizing the inspection of D1 by third parties and translation thereof
- Sworn declaration by Dr. Fabio Rezzonico and signede copy of D1
- Copy of a presentation by Dr. Veronica Albertini given on 22.03.2012 - authorization of a foreign promotion agent (Success plus beauty consultation & training Co. limited) - picture with foreign promotion agent
- Sworn declaration by Dr. Veronica Albertini & ILLOUZ Y-G. ET AL: 'Adipose Stem Cells and Regenerative Medicine', 2011, SPRINGER VERLAG, BERLIN
- Definition from Wiktionary concerning "Lipoaspirate"
- Summary od EPO Case Law concerning University Thesis
- Declaration by Dr. Gianni Soldati
- Declaration by Dr. Richard D'Agostino
- Declaration by Prof. Roberto Taramelli
- Case Law review on oral disclosures
- Contemporary notes of Dr. Richard D'Agostino (not open to public)

## Description

Stem cells are gaining a growing interest in cosmetic and medical practice, in connection with their capacity to generate new biological tissues applicable to patients who, for various reasons, have lost these tissues or the capacity to regenerate them.

In particular, adult stem cells, e.g. those from lipid or myeloid origin, have attracted particular interest, due to their more controllable potency and also for being exempt from the ethic restrictions applicable to embryonic ones.

A typical field of use for stem cells of lipid origin is that of tissue filling for cosmetic purposes: here, the patient requiring treatment donates a part of his own lipid tissue (lipoaspirate); this is processed by a laboratory recovering the purified cell fraction, the latter being injected back into the patient in body areas requiring filling. The cell fraction obtained is called Stromal Vascular Fraction (SVF) and represents the total number of nucleated cells extracted from the adipose tissue. Typically, 10 to 20% of these cells are stem cells, called also mesenchimal stem cells. They are pluripotent and capable of repairing or regenerating several types of human tissues. The use of autologous lipid tissue avoids the risk of immune reactions and rejection of the tissue. Furthermore, these extracted cells can then be stored frozen for long periods in liquid nitrogen at disposal of the donor for further treatments including the use of stem cells in human stem cell therapies.

Processes to prepare purified cells fractions of lipid tissue origin are known in the art.

These generally comprise the recovery of the original raw lipid material, the selective extraction of the stem cell component, the elimination of the exhausted lipid matrix, and the final washing, purification and preparation of the stem cells fraction in a convenient concentration and volume. These processes, involving the passage of the stem cells-containing material through a number of pipettes, tubes, beckers, etc. involve a considerable risk of bacterial contaminations and/or material loss: therefore they require very strict procedures as regards of sterility of the environment and of the different materials used, multiple washings to recover possibly adhered material, all adding up to the overall cost of the process. The multiple container transfers also increase the risk of inadvertent exchange of samples from different patients, exposing the final stem-cell receiver to the risk of non-autologous transfusion.

The above procedure also requires a strict collaboration and understanding between the operator collecting the lipid raw material and the laboratory isolating the stem cells. Sometimes the raw material is sent in non-optimal containers (e.g. too large, not properly sealed, wrongly packaged, in not optimal amounts, etc.): in all these cases, the laboratory is forced to work with a sub-optimal starting sample, which may affect the quality of the final product.

Aim of the invention is to provide an improved process to prepare stem cell fractions of lipid tissue origin, which is safer for the patient and more expedite for the process operator.

A further aim is to simplify / standardize the interface and cooperation between the operators collecting the lipid raw material, and those charged with the stem cell isolation.

A further aim is to reduce the number of steps and manipulations involved in processes for producing purified cell fractions.

A further aim is to make more rapid and effective those medical/cosmetic procedures involving the use of stem cells.

### SUMMARY

The present invention relates to a process to obtain a stem cell fractions of lipid origin, essentially based on the steps of:
(a) receiving a sample of lipid tissue containing stem cells;
(b) washing the sample obtained in step (a) with a suitable aqueous buffer;
(c) incubating the sample obtained in step (b) with an enzyme capable to digest the lipid tissue and extract therefrom the stem cells;
(d) recovering the aqueous phase from the product obtained in step (c);
(e) purifying the aqueous phase obtained in step (d);
(f) titrating the aqueous phase obtained in step (e) and optionally diluting it to obtain a final stem cell fraction with desired concentration and volume.

One essential feature of the invention consists in that the stem cells-containing material is treated within the same collecting device (herein referred as "single collecting device" or "SCD"), throughout at least the steps: (a), (b), and (c) of the process described herein. The use of the SCD avoids contact of the treated material with the external atmosphere, reduces to a minimum the risk of contamination and the loss of active material linked to multiple container transfers, and simplifies the overall manipulations required to obtain purified stem cell fractions.

The SCD is a sterile container capable to draw and release a liquid, i.e. a syringe. Said SCD may have a large filling volume (e.g.20 to 100 mL), to allow a large scale harvesting of stem cells from the corresponding lipid material. It is preferably transparent or semi-transparent, with one mark indicating the optimal filling volume, and/or areas aimed at writing or labelling, to identify the sample source.

As will be evident from the description, said SCD fulfils the functions of a collecting device (like a pipette), a phase separator, and process reactor, depending on the particular process step involved; all these functions are thus advantageously performed without transferring the sample from one container to another, and/or contacting it with the external atmosphere.

Said SCD is essentially used throughout steps (a),(b) and (c) of the process: however, if desired, the SCD can be maintained also throughout one or more of steps (d),(e),(f), with the same functions and advantages described above.

Based upon the above premises, the process of the invention comprises the following steps:
(a) receiving, in said SCD, a sample of lipid tissue containing the stem cells;
(b) in said SCD, washing the sample of step (a) with a suitable aqueous buffer;
(c) in said SCD, incubating the sample of step (b) with an enzyme capable to digest the lipid tissue and extract therefrom the stem cells;
(d) recovering the aqueous phase from the product of step (c);
(e) purifying the aqueous phase obtained in step (d);
(f) titrating the aqueous phase obtained in step (e) and, if necessary, diluting it to a final stem cell fraction with desired concentration and volume.

### DETAILED DESCRIPTION OF THE INVENTION

### Step (a):

In this step, a sample of lipid tissue containing stem cells, contained into said SCD, is received by an operator.

Lipoaspirates are typical lipid tissue samples. The sample must be liquid or at least fluid for the purpose of the present process; insufficiently fluid materials can be rendered such by further homogenisation and/or addition of liquid media, e.g. buffered solutions.

The expression "receiving" in step (a) accounts for the fact that the operator "receives" the lipid sample, collected by someone else, and processes it per steps (b)-(f).

The present process is particularly advantageous in that it removes a primary cause for contamination occurring in known processes where the lipoaspirate is collected into a first container, stored, sent to an external laboratory and then transferred into a suitable reactor: all these transfers/manipulations involved a contact of the sample with the external atmosphere, with the connected risk of contamination, along with an inevitable percent of product loss. Such disadvantages and risks are now minimized by the present process.

The use of the said SCD is further advantageous in that it provides the initial operator, i.e. the one collecting the lipid sample, with a standardized container, suitably adapted for the further processing from the point of view of filling volume, void volume, air-tightness, packaging material, etc.

The following steps can be performed immediately after step (a); alternatively the partially filled SCD is stored for a certain time, at conditions maintaining the viability of the stem cells, until the time of further processing as per steps (b)-(f).

### Step (b).

In this step, the drawn lipid sample from step (a) is washed inside the SCD, with an aqueous buffer solution.

To do so, the partially filled SCD from step (a) is operated to draw a volume of a buffer solution, which mixes with the lipid tissue sample present inside the SCD; homogeneous mixing of the two phases can be facilitated e.g. by applying vibrations /shaking to the SCD. The used buffer solution is a stem cell-compatible one, typically a PBS buffer supplemented with a calcium and/or magnesium salts useful as enzyme nutrients; the volume ratio of buffer to lipid tissue is e.g. from about 0.5:1.5 to about 1.5:0.5; preferably it is about 1:1.

After said mixing/homogenizing, the SCD is kept still until the two phases (lipid and aqueous) separate. Then the SCD is then operated to eject the aqueous phase while retaining the lipid phase: being the SCD a syringe, this can be done by orienting it in downward (needle-down) position: this causes the lipid phase to move in the upper section of the syringe, distal from the needle, while the aqueous phase aggregates into the opposite section, proximal to the needle: in this position, a pressure on the syringe plunger causes the aqueous phase to be ejected from the needle; the pressure is suitably maintained until the water/lipid interface reaches the needle: at this point the aqueous phase (to be discarded) is substantially eliminated, and the syringe contains only the lipid phase, upon which the next step is to be performed.

The above-described washing step can be repeated more times, e.g. one or two, until the desired degree of washing of the lipid phase is reached.

### Step (c)

In this step the washed lipid phase from step (b) is incubated in the SCD with an enzyme capable to extract the stem cells from the lipid material containing them.

To perform this step, the SCD is operated to further draw an aliquot of a liquid medium containing said enzyme. The enzyme is typically a liberase, The liquid medium is typically a buffer, preferably a PBS buffer optimized for enzyme activity, in particular supplemented with calcium and/or magnesium salts; the liquid medium has a known enzymatic titre, allowing the operator to draw a desired and reproducible amount of the enzyme.

The thus filled SCD, optionally inserted within a sealed envelope, is then placed into an incubator, typically a temperature-controlled oven provided with an oscillating tray. Prior to incubation, the SCD is preferably agitated to homogenize the content; mixing is then continued within the incubator, by the oscillation movement of the tray.

The incubator may be operated under the following non-exhaustive conditions: incubation time 20-80 minutes, preferably 30-60 minutes, most preferably 45 minutes; temperature of 30-45 °C, preferably at 37°C; agitation: 1-5 rpm, preferably 2 or 3 rpm.

### Step (d)

In this step the enzyme reaction is blocked, the lipid phase is eliminated, and the aqueous phase (containing the stem cells, liberated by the enzyme) is recovered for further processing per steps (e)-(f).

To perform this step, the SCD is removed from the incubator and extracted from the (optionally used) envelope; the incubated suspension is mixed with an aliquot of an enzyme-inactivating solution, for example a buffered albumin solution at a suitable concentration, e.g. 1% by weight.

A suitable mode of mixing the two liquids consist in drawing the inactivating solution into the SCD, agitating the SCD to obtain complete homogenization, keeping the SCD still until the lipid and aqueous phases separate, and recovering the aqueous phase.

Recovery of the aqueous phase can be done by ejecting it from the SCD (ejection mode), or, alternatively, by retaining it into the SCD (retention mode).

The "ejection mode" can be performed by orienting the syringe in the downward (needle-down) position: this causes the lipid phase to move in the upper section of the syringe, distal from the needle, while the aqueous phase aggregates into the opposite section, proximal to the needle: in this position, a pressure on the syringe plunger causes the aqueous phase to be ejected from the needle; the pressure is suitably maintained until the water/lipid interface reaches the needle: at this point the aqueous phase (to be collected for further processing per steps (e)-(f)) is substantially ejected from the syringe; the latter contains the stem cell-depleted lipid phase which can now be ejected separately and eliminated.

Alternatively to the ejection mode, the "retention mode" can be performed by orienting the syringe in the upward (needle-up) position: this causes the lipid phase to move in the section of the syringe, proximal to the needle, while the aqueous phase aggregates into the opposite section, distal from the needle: in this position, a pressure on the syringe piston causes the lipid phase to be ejected from the needle; suitable means can be used to avoid the dispersion of the liquid ejected from the up-oriented syringe: for example, prior to ejection, the needle may be inserted through the rubber stopper of a flask, into which the ejected liquid is then collected. The pressure is suitably maintained until the water/lipid interface reaches the needle: at this point the lipid phase (to be discarded) is substantially ejected from the syringe; the latter contains the aqueous phase meant for further processing per steps (e)-(f).

At the end of step (d) the aqueous phase is recovered from the SCD and treated separately, unless the SCD has a shape and consistence allowing it to be centrifuged: in this case, the subsequent process steps can also be performed into the SCD, adding further protection/simplification to the overall process.

The emptied SCD, if not adapted for centrifugation, is preferably washed one or more times with an appropriate solution (preferably the inactivating solution described above) to recover possible stem cells adhering to its surfaces, and all the resulting aqueous phases are pooled for the further processing according to steps (e)-(f))

### Step (e)

In this step, the (pooled) aqueous phases from step (d) are purified from possible soluble/insoluble impurities derived from the original lipid sample. Purification is generally obtained by centrifugation, elimination of the supernatant, re-suspension of the pellet, filtration. Centrifugation can be generally performed: at a speed of 300-500 G, preferably 350-450 G, more preferably at 400 G; for a time of 1-10 minutes, preferably 3-7 minutes, more preferably for 5 minutes.

After eliminating the supernatant, the re-suspension of the pellet can be performed by using a suitable buffer (e.g. a PBS buffer) or the inactivating solution described above.

The above centrifugation and re-suspension can be repeated one or more times to increase purification of the particulate (stem cell) fraction from water soluble impurities.

The (finally) re-suspended pellet is then filtered one or more times, to eliminate particulate impurities being oversize with respect to the stem cell fraction. To do so, the suspended pellet is filtered through a membrane with an appropriate pore size, e.g. 80-120 µm, preferably about 100 µm, retaining the oversized particulate material, and allowing the (lower sized) stem cells to pass in the filtrate. The resulting liquid can be filtered again with progressively finer filters, e.g. 60-80 µm, preferably about 40 µm, to allow a finer elimination of the oversized particulate. The finally filtered liquid, containing the purified stem cells is further processed per step (f).

### Step (f)

In this step, the purified liquid from step (e) is titrated and then diluted to obtain a final stem cell fraction with desired concentration and volume.

Titration can be done by withdrawing a precise volume of the liquid of step (e) (e.g. 50 µL) and subjecting it to a stem cell count by means of a suitable counting apparatus, typically a FACS with optimized gates to obtain Adipose-Derived Mesenchimal Stem Cells counts; alternatively, the stem cell content can be assessed indirectly by means of other instruments e.g. a haemocytometer: the latter counts the total number of nucleated cells which, at this stage of the process, are found to be stem cells by 10-20%. Preferably, two or more readings are taken and averaged, for a higher precision.

Based upon its known titre, the liquid from step (e) can, if necessary, be diluted to an appropriate concentration. Dilution can be performed by using a suitable buffer (e.g. a PBS buffer) or the inactivating solution described above. The final concentration value is chosen in function of the desired level of potency of the final stem cells fraction; useful, non limitative stem cell concentration values (expressed as total nucleated cells) are from 10⁸ to 10⁴ cells/ml, preferably 10⁷ to 10⁵ cells/ml, more preferably about 10⁶ cells/ml.

The final stem cell fraction can then be packaged in a suitable container (e.g. mini-syringe) as a unit with an appropriate volume, e.g. 1 mL; the final volume is chosen to be compatible and handy with the site of administration (e.g. wrinkle filling, tissue reconstruction, etc.) of the final stem cell fraction.

The final stem cell fraction is preferably used as soon as possible or, alternatively, it is stored in suitable conditions of sterility and temperature, until the time of use.

It can be used for any application in which stem cells of lipid origin are useful. Non limitative examples are in the field of aesthetic or reconstructive treatments, in particular tissue filling, wound healing, tissue or organ reconstruction.

A suitable, non-exhaustive procedure in accordance with the present invention is described as follows.

### EXAMPLE 1

### 1.1 Washing with PBS Ca/Mg buffer

A 100 mL syringe (SCD), containing 50 mL of raw lipoaspirate, was filled with 50 ml of a standard Dulbecco's PBS buffer containing calcium and magnesium salts. The syringe (SCD) was inverted 10 times to homogenize the content, and then kept still in vertical position (needle down) for about 5 minutes, allowing the aqueous and lipid phases to separate. The syringe plunger was then pressed to eject the entire lower (aqueous) phase, retaining the lipid phase in the syringe.

The washing procedure was repeated, and the ejected aqueous phases were discarded.

### 1.2. Incubation with liberase

The syringe (SCD) resulting from step 1.1 containing the washed lipid phase, was operated to draw the enzymatic reagent to reach a concentration of 0.28 Wünsch Units/ml. The enzymatic reagent was prepared in advance from a 0.028 Wünsch Units/µL mother solution of liberase ("MNP-S" in diluted in Dulbecco's PBS containing Calcium and Magnesium), diluted 1:500 v/v with the PBS Ca/Mg buffer described above.

The so filled syringe (SCD) was further operated to draw 20 ml of sterile air, inverted 10 times to homogenize the content; sealed in an envelope and put in an incubator with oscillating tray, preheated at 37°C. Incubation was performed at 37°C for 45 minutes, with oscillation at 3 rpm.

### 1.3. Inactivation of liberase

After expiry of the incubation time, oscillation was interrupted, the syringe (SCD) removed from the incubator, released from the envelope, and added with an equal volume of a 1 % wt albumin solution in Dulbecco's PBS. The so filled syringe (SCD) was further operated to draw 5 ml of sterile air, and inverted 10 times to homogenize the content; and then kept still in vertical position (needle down) for about 5 minutes, allowing the aqueous and lipid phases to separate.

### 1.4. Recovery of the aqueous phase

The plunger of the syringe (SCD) was pressed to eject the entire lower phase (aqueous phase): this phase, containing the stem cells, was recovered into a centrifuge tube (marked with "1^{st} recovery"). The lipid phase remaining in the syringe (SCD) was ejected apart and discarded; the empty syringe (SCD) was then washed with 1% albumin solution, according to the procedure described at point 1.3, and the washing solution was collected in a further centrifuge tube (marked with "2^{nd} recovery")

### 1.5. First centrifugation and pellet resuspension

The two tubes obtained at point 1.4, (1^{st} and 2^{nd} recovery) were centrifuged at 400 G for 5 minutes at 20°C. All supernatants were removed; the pellets in the 2^{nd} recovery tubes were resuspended manually with 20 mL of a 1% albumin in PBS; the resulting suspension was added to the 1^{st} recovery tubes and used to resuspend the pellet therein present.

### 1.6. Filtration, second centrifugation and pellet resuspension

The suspension obtained at the end of point 1.5. was filtered via two subsequent steps, using filters with pore size of 100 and 40 µm, respectively. The filters were eliminated. The finally obtained liquid, containing the stem cells, was centrifuged at 400 G, for 5 minutes at 20°C. The supernatant was eliminated and the pellet was resuspended with 5 ml of a 1% albumin solution in PBS.

### 1,7. Titration

Using a 1 mL pipette, two samples of 50 µL were collected from the filtered suspension obtained at point 1.6 (Stromal Vascular Fraction, SVF) and inserted for reading in a haemocytometer. The two readings (expressed as total nucleated cells i.e. WBC/mL,) were averaged, to obtain an accurate titre of the solution.

A direct count of the stem cells was also performed on a 1 mL sample of the solution obtained at point 1.6: the sample was centrifuged at 1300 G for 5 minutes; the supernatant was eliminated and the pellet resuspended with 0.440 µL of FACS buffer (PBS + 1% Human Serum); the solution, incubated for 20 minutes with suitable antibodies and added with Versalyse and Stem-cell count solution, was then read on a Navios cytofluorimeter, assessing the number of stem cells present in the sample and, therefrom, the relevant stem cell concentration.

### 1.8 Dilution to standard concentrations and packaging

Based on the titre measured at point 1.7., the remainder of the solution obtained at point 1.6. was diluted with a 5% albumin solution in PBS to concentrations and volumes convenient for practical stem cell treatments. A useful range of concentrations is between 0.5 and 3x10⁶ WBC/mL. The resulting solution, was finally subdivided into 1 mL sterile syringes, which were then packaged and sealed for expedition and delivery to the final user.

## Claims

1. Process to prepare stem cells fractions of lipid tissue origin, comprising the steps of:
(a) receiving a sample of lipid tissue containing the stem cells;
(b) washing the sample of step (a) with a suitable aqueous buffer;
(c) incubating the sample of step (b) with an enzyme capable to digest the lipid tissue and extract therefrom the stem cells;
(d) recovering the aqueous phase from the product of step (c);
(e) purifying the aqueous phase obtained in step (d);
(f) titrating the aqueous phase obtained in step (e) and, if necessary, diluting it to obtain a final stem cell fraction with desired concentration and volume
wherein the stem cells-containing material is treated within a syringe throughout at least the steps: (a), (b), and (c), said syringe performing the functions of collecting means, phase separator and process reactor.

2. Process according to claim 1, wherein the syringe has a filling volume comprised between 20 and 100 mL.

3. Process according to claims 1-2, wherein the syringe is provided with one or more marks to indicate the optimal filling volume(s), and/or with areas for writing or labelling.

4. Process according to claims 1-3, wherein the lipid material is a lipoaspirate.

5. Process according to claims 1-4, wherein the step (a) and (b-d) respectively, are performed by two different operators at locations remote from each other.

6. Process according to claims 1-5, wherein in step (b) the buffer is a PBS buffer supplemented with enzyme nutrients.

7. Process according to claims 1-6, in which the steps (b) and/or (c) include orienting the syringe downwards (needle down) or upwards (needle up), followed by ejecting the phase proximal to the needle.

8. Process according to claims 1-7, wherein in step (c) the enzyme is a liberase, and the incubation is performed for 20 to 80 minutes, at a temperature from 30 to 45°C.

9. Process according to claims 1-8, wherein in step (d), the incubated mixture is mixed with an albumin-containing solution, then the lipid phase is discarded and the aqueous phase is recovered for the subsequent process steps.

10. Process according to claims 1-9, where the stem cells-containing material is further maintained within the said syringe during one or more of the steps (d)-(e).

11. Process according to claims 1-10, wherein purification in step (e) is performed by centrifugation(s) and/or filtration(s).

12. Process according to claims 1-11, wherein the final stem cell fraction is formulated as one or more units of 1-5 mL, with a total nucleated cell concentration comprised between 10⁸ to 10⁴ cells/mL.

## Patentansprüche

1. Verfahren zur Herstellung von Stammzellfraktionen aus Fettgewebe als Herkunft, umfassend die Schritte:
(a) Erhalten einer Probe von Fettgewebe, die die Stammzellen enthält;
(b) Waschen der Probe aus Schritt (a) mit einem geeigneten wässrigen Puffer;
(c) Inkubieren der Probe aus Schritt (b) mit einem Enzym, dass fähig ist, das Fettgewebe zu verdauen und daraus die Stammzellen zu extrahieren;
(d) Gewinnen der wässrigen Phase aus dem Produkt von Schritt (c);
(e) Reinigen der in Schritt (d) erhaltenen wässrigen Phase;
(f) Titrieren der in Schritt (e) erhaltenen wässrigen Phase und, falls notwendig, Verdünnung, um eine endgültige Stammzellfraktion mit der gewünschten Konzentration und Volumen zu erhalten
wobei das Stammzellen enthaltende Material innerhalb einer Spritze behandelt wird, während mindestens der Schritte: (a), (b) und (c), wobei die Spritze die Funktionen der Sammlung, der Phasentrennung und des Prozessreaktors ausführt.

2. Verfahren nach Anspruch 1, wobei die Spritze ein Füllvolumen zwischen 20 und 100 mL umfasst.

3. Verfahren nach den Ansprüchen 1-2, wobei die Spritze mit einer oder mehreren Markierungen versehen ist, um die optimale Füllmenge(en) anzuzeigen, und/oder mit Flächen zum Schreiben oder Kennzeichnen.

4. Verfahren nach den Ansprüchen 1-3, wobei das Lipid-Material ein Lipoaspirate ist.

5. Verfahren nach den Ansprüchen 1-4, wobei der Schritt (a) und (b-d) jeweils von zwei verschiedenen Anwendern an voneinander entfernten Standorten durchgeführt werden.

6. Verfahren nach den Ansprüchen 1-5, wobei in Schritt (b) der Puffer ein PBS Puffer ist ergänzt mit Enzymnährstoffen.

7. Verfahren nach den Ansprüchen 1-6, in dem die Schritte (b) und/oder (c) das Ausrichten der Spritze nach unten (Nadel unten) oder nach oben (Nadel oben) beinhaltet, gefolgt von dem Ausstoßen der Phase proximal zu der Nadel.

8. Verfahren nach den Ansprüchen 1-7, wobei in Schritt (c) das Enzym eine Liberase ist, und die Inkubation für 20 bis 80 Minuten, bei einer Temperatur von 30 bis 45 C°, durchgeführt wird.

9. Verfahren nach den Ansprüchen 1-8, wobei in Schritt (d) die inkubierte Mischung mit einer Albumin-haltigen Lösung gemischt wird, dann die Lipidphase verworfen wird und die wässrige Phase für die nachfolgenden Prozessschritte wiederhergestellt wird.

10. Verfahren nach den Ansprüchen 1-9, wobei das stammzellenhaltige Material während einem oder mehrerer Schritte (d)-(e) weiter innerhalb der Spritze bleibt.

11. Verfahren nach einem der Ansprüche 1-10, wobei die Reinigung in Schritt (e) durch Zentrifugation(en) und/oder Filtration(en) durchgeführt wird.

12. Verfahren nach den Ansprüchen 1-11, wobei die endgültige Stammzellfraktion als eine oder mehrere Einheiten von 1-5 mL formuliert ist, mit einer kernhaltigen Gesamtzellkonzentration, umfassend zwischen 10⁸ bis 10⁴ Zellen/mL.

## Revendications

1. Procédé de préparation de fractions de cellules souches ayant pour origine du tissu lipidique, comprenant les étapes de :
(a) recevoir un échantillon de tissu lipidique contenant les cellules souches;
(b) laver l'échantillon de l'étape (a) avec un tampon aqueux approprié;
(c) incuber l'échantillon de l'étape (b) avec une enzyme capable de digérer le tissu lipidique et en extraire les cellules souches;
(d) récupérer la phase aqueuse du produit de l'étape (c);
(e) purifier la phase aqueuse obtenue à l'étape (d);
(f) titrer la phase aqueuse obtenue à l'étape (e) et, si nécessaire, la diluer pour obtenir une fraction de cellules souches finale avec la concentration et le volume souhaités,
dans lequel le matériau contenant les cellules souches est traité au sein d'une seringue au moins tout au long des étapes: (a), (b) et (c), ladite seringue réalisant les fonctions de moyen de recueil, séparateur de phase et réacteur opératoire.

2. Procédé selon la revendication 1, dans lequel la seringue a un volume de remplissage compris entre 20 et 100 ml.

3. Procédé selon les revendications 1 et 2, dans lequel la seringue est pourvue d'une ou plusieurs marques pour indiquer le(s) volume(s) de remplissage optimal(ux) et/ou de zones pour écrire ou étiqueter.

4. Procédé selon les revendications 1 à 3, dans lequel le matériau lipidique est un produit de liposuccion.

5. Procédé selon les revendications 1 à 4, dans lequel les étapes (a) et (b à d), respectivement, sont réalisées par deux opérateurs différents en des emplacements à distance l'un de l'autre.

6. Procédé selon les revendications 1 à 5, dans lequel, à l'étape (b), le tampon est un tampon PB S additionné de nutriments enzymatiques.

7. Procédé selon les revendications 1 à 6, dans lequel les étapes (b) et/ou (c) incluent l'orientation de la seringue vers le bas (aiguille vers le bas) ou vers le haut (aiguille vers le haut) suivie de l'éjection de la phase proximale à l'aiguille.

8. Procédé selon les revendications 1 à 7, dans lequel, à l'étape (c), l'enzyme est une libérase et l'incubation est réalisée pendant 20 à 80 minutes à une température de 30 à 45°C.

9. Procédé selon les revendications 1 à 8, dans lequel, à l'étape (d), le mélange incubé est mélangé à une solution contenant de l'albumine, puis la phase lipidique est jetée et la phase aqueuse est récupérée pour les étapes opératoires subséquentes.

10. Procédé selon les revendications 1 à 9, dans lequel le matériau contenant les cellules souches est en outre maintenu au sein de ladite seringue au cours d'une ou plusieurs des étapes (d) et (e).

11. Procédé selon les revendications 1 à 10, dans lequel la purification à l'étape (e) est réalisée par centrifugation(s) et/ou filtration(s).

12. Procédé selon les revendications 1 à 11, dans lequel la fraction de cellules souches finale est formulée en tant qu'une ou plusieurs unités de 1 à 5 ml avec une concentration de cellules nucléées totale comprise entre 10⁸ et 10⁴ cellules/ml.
